(19) Europäisches Patentamt
European Patent Office
Office européen des brevets

(11) Publication number: **0 003 753**
**B1**

# (12) EUROPEAN PATENT SPECIFICATION

(45) Date of publication of patent specification: 07.04.82

(51) Int. Cl.[3]: C 07 C 45/57, C 07 D 317/26, C 07 D 319/06

(21) Application number: 79100233.0

(22) Date of filing: 29.01.79

## (54) Process of hydroformylating cyclic acrolein acetals.

(30) Priority: 21.02.78 US 880222
17.11.78 US 960656

(43) Date of publication of application:
05.09.79 Bulletin 79/18

(45) Publication of the grant of the patent:
07.04.82 Bulletin 82/14

(84) Designated Contracting States:
BE DE FR GB IT NL

(56) References cited:
DE - A - 2 541 438
FR - A - 2 346 311
FR - A - 2 351 935
US - A - 4 024 197
US - A - 4 052 401

(73) Proprietor: E.I. DU PONT DE NEMOURS AND COMPANY
Legal Department 1007 Market Street
Wilmington Delaware 19898 (US)

(72) Inventor: Cumbo, Charles Carmen
1410 Bucknell Road Green Acres
Wilmington Del. 19803 (US)

(74) Representative: Abitz, Walter, Dr.-Ing. et al,
Abitz, Morf, Gritschneder P.O. Box 86 01 09
D-8000 München 86 (DE)

EP 0 003 753 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process of hydroformylating cyclic acrolein acetals

## DESCRIPTION

*Technical field*

This invention relates to an improved process for the hydroformylation of cyclic acrolein acetals to the corresponding aldehydes in the presence of carbon monoxide, hydrogen, rhodium-complex catalyst and triphenyl phosphite ligand. More specifically, this invention relates to the hydroformylation of cyclic acrolein acetals to the corresponding aldehydes in the presence of carbon monoxide, hydrogen, 1—50 ppm of rhodium in a rhodium-complex catalyst based on the acetal and excess triphenyl phosphite ligand.

*Background art*

Hydroformylation reactions wherein various olefins are reacted with carbon monoxide and hydrogen to form corresponding aldehydes are known in the art. For example, U.S. Patent 3,527,809 discloses the hydroformylation of alpha olefinic compounds at certain temperatures and gas pressures wherein the partial pressure of carbon monoxide is about 25—75% of the total pressure, in the presence of certain rhodium-containing complexes and organo phosphorus ligands to obtain a high linear to branched chain aldehyde isomer ratio. Rhodium concentrations as low as $1 \times 10^{-6}$ mole per mole of alpha olefinic feed are disclosed as operable but are said to be not particularly desirable since the reaction rate appears to be too slow and thus not commercially attractive. A rhodium concentration of $1 \times 10^{-5}$ up to $5 \times 10^{-2}$ mole per mole of alpha olefinic feed is disclosed as desirable. The disclosure and teaching is directed to hydroformylation with ligands that may be a tertiary organo phosphorus compound, a tertiary organo arsenic compound or a tertiary organo antimony compound. The disclosure equates ligands containing phosphorus, arsenic and antimony and also equates trialkyl phosphite, tricycloalkyl phosphite, triaryl phosphite and triaryl phosphine ligands.

*Disclosure of the invention*

Now it has been found that the hydroformylation reaction of cyclic acrolein acetals can be advantageously conducted at rhodium concentrations of 1—50 ppm based on the starting acetal under the conditions of the process of this invention. Accordingly, the process of this invention is an improvement in the hydroformylation of cyclic acrolein acetals to the corresponding aldehydes in the presence of carbon monoxide, hydrogen, rhodium complex catalyst and an organo phosphorus ligand wherein the improvement comprises conducting said process in the presence of an amount of rhodium complex catalyst equivalent to from 1—50 ppm of rhodium based on the cyclic acetal, a ratio of $H_2$ to CO of from 1:1—20:1, a CO partial pressure of from 34,5—137,9 kPa, 1—3% by weight based on the cyclic acetal of excess triphenyl phosphite ligand and at a temperature of from 100—115°C.

The process of the present invention maximizes the ratio of linear to branched chain aldehyde and minimizes the rhodium level required for the process without the reaction rate decrease that occurs with other ligands. At the preferred rhodium level of 1—10 ppm, the economic incentive to recover rhodium from the process is not sufficient to justify recovery of the rhodium.

The cyclic acrolein acetals of this invention are defined as cyclic acrolein acetals of alkane diols and are represented by the formulae

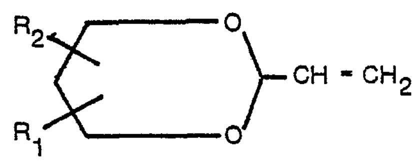

or

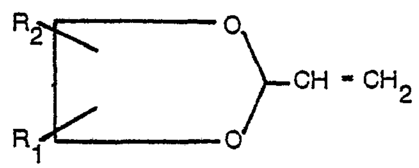

where $R_1$ and $R_2$ are selected from H and alkyl groups of 1—4 carbon atoms. Preferably $R_1$ and $R_2$ are selected from hydrogen and alkyl groups of 1—2 carbon atoms. $R_1$ and $R_2$ can be the same or different alkyl groups.

Representative examples of the cyclic acrolein acetals of this invention include 2-vinyl-5-methyl-1,3-dioxane (VMD), 2-vinyl-4-methyl-1,3-dioxane (VMD), 2-vinyl-5,5-dimethyl-1,3-dioxane, 2-vinyl-1,3-dioxane, 2-vinyl-1,3-dioxolane, 2-vinyl-4-methyl-1,3-dioxolane and 2-vinyl-4,5-dimethyl-1,3-dioxolane.

The cyclic acrolein acetals of this invention may be prepared as taught in U.S. Patent 4,024,159.

In the process of the present invention linear aldehydes of 80 mole percent or more, preferably 85 mole percent or more, based on the combined linear and branched-chain aldehydes are obtained. The attainment of linear aldehydes amounting to 80 mole percent or more of the aldehydes produced is achieved together with shorter reaction times than that attained when other ligands are substituted for the ligand of the invention.

Rhodium, the catalyst, is present during the process of the invention in the form of a

complex catalyst with carbon monoxide and triphenyl phosphite ligand.

"Rhodium complex catalyst" as used herein means a coordination compound formed by the union of one or more electronically rich molecules or atoms capable of independent existence with one or more electronically poor molecules or atoms, each of which are also capable of independent existence. Said catalyst is a combination of rhodium, carbon monoxide and ligand and can be formed from rhodium carbonyl and the ligand indicated herein. The rhodium complex catalyst can be formed in situ when rhodium in the form of $Rh_6(CO)_{16}$ is added to the hydroformylation reaction containing the ligand of the invention or it can be prepared first and then added to the reaction mixture.

The rhodium complex catalyst of the present invention is a homogeneous catalyst solution comprising rhodium and ligands complexed with carbon monoxide through coordinate bonding. The rhodium catalyst may be employed in a finely divided form and dispersed in and throughout the reaction mixture, or it may be employed in a more massive state, either in essentially the pure state or supported upon or carried by an inert or catalytically active support or carrier material, such as pumice kieselguhr, diatomaceous earth, clay, alumina, charcoal, carbon, or the like, and the reaction mixture contacted therewith as by flowing the mixture over or through a bed of the catalyst or according to other methods that are known in the art.

The process of the present invention is conducted at a pressure of carbon monoxide of from 34,5—137,9 kPa. At said carbon monoxide pressures, the pressure of hydrogen would range from 34,5—2757,6 kPa. When the pressure of carbon monoxide is above 137,9 kPa, the ratio of linear to branched chain aldehyde is less than 80%. When the carbon monoxide partial pressure is below 5 psia, the gas composition is such that too much hydrogenation at the expense of hydroformylation will occur, thereby inflicting a yield loss on the aldehyde formed. Additionally, the pressure range of the process of the present invention at the rhodium range permits satisfactory reaction rates. Reaction rate is first order to the hydrogen concentration in the liquid reaction medium. The hydrogen concentration in the liquid is proportional to the partial pressure of hydrogen. Therefore, the reaction rate is first order to the hydrogen partial pressure.

Surprisingly the hydroformylation process of the present invention results in a significantly faster reaction rate than that where ligands other than those of the present invention are used. The process of the present invention achieves this at rhodium levels of from 1—50 ppm rhodium based on the starting acetal. At the preferred rhodium level of 1—10 ppm, commercial operation at the conditions of this invention are not only achieved at faster reaction rates than with other ligands, but are also economically feasible without a rhodium recovery operation based on the starting cyclic acetal.

The process of this invention not only results in faster reaction rates than that where other ligands are used, but also results in a ratio of linear to branched chain aldehyde of 80% or more. The reaction rates with the ligand of the invention vary, but generally are in the range of, e.g., about 1.5—80 hours based on 100% conversion of VMD.

The ligand of the present invention is critical and is limited to triphenyl phosphite. Under the present conditions reactions with other ligands are not as fast as they are with triphenyl phosphite. Thus, the use of triphenyl phosphite under the conditions of this invention result in a faster reaction rate than other ligands.

The excess ligand concentration in the process of this invention of from 1—3% by weight based on the cyclic acetal is critical. An excess ligand concentration of below 1 % by weight reduces the ratio of linear to branched chain aldehyde below 80%. An excess ligand concentration above 3% by weight seriously reduces the rate of reaction. What is meant by excess ligand concentration is that amount of ligand in excess over that required to form the rhodium complex catalyst.

The temperatures at which the process of this invention are conducted may range from 100—115°C, preferably 105—110°C. At a temperature above 115°C, the formation of high boilers increases above desirable levels.

The present process may be operated batchwise or continuously in conventional equipment having agitation means sufficient to provide uniform mixing of reactants.

The process of the present invention is useful in the preparation of cyclic acrolein acetal aldehydes that are useful intermediates for the production of 1,4-butanediol which is useful as a solvent and a resin intermediate.

The gas mole ratio of $H_2$:CO may be in the range of from 1:1—20:1. However, at lower rhodium levels, the gas mole ratio is generally from 2:1—20 :1. For example, at the preferred level of 1—10 ppm rhodium the required $H_2$:CO ratio is 2:1—20:1.

BEST MODE

The best mode for carrying out the invention is illustrated in Example 1.

The invention is further illustrated by the examples that follow where all parts and percentages are by weight unless otherwise indicated.

Example 1

A 300 cc Magne-Drive Autoclave (Autoclae Engineers, Inc.) equipped with a turbine-type agitator with a hollow shaft was charged with (a) 77.0 g (0.6 mole) of 2-vinyl-4-methyl-1,3-dioxane (VMD) that was treated with $LiAlH_4$ to

remove water, 1,4-butanediol and acrolein present therein and then vacuum distilled, (b) 1.57 g (0.005 mole) of excess purified triphenyl phosphite (2% based on the VMD) and (c) 0.00053 g (5.0 x $10^{-7}$ moles) of hexarhodium hexadecacarbonyl [$Rh_6(CO)_{16}$]. The rhodium concentration based on the VMD was 4 ppm. The autoclave was flushed with a 5:1 hydrogen/carbon monoxide mixture and then pressurized to 434,4 kPa (partial pressure of carbon monoxide of 72,4 kPa and held constant at that value. The gas mixture above the liquid in the autoclave was maintained at a constant ratio (5:1 $H_2$/CO) by purging the gas at a rate of 750 cc/minute. The contents were heated by an electric oven to 105°C and agitated at 1,500 rpm. The progress of the reaction was monitored by taking aliquots every hour and analyzing by g.l.p.c. (gas liquid phase chromotography) using a 0.32 cm x 304.8 cm column packed with 10% Triton X 305 on Chromasorb W. After 6 hours, 70% of the VMD was converted to aldehyde (92.2%) and 2-ethyl-4-methyl-1,3-dioxane (7.8%). Of the aldehyde product, 86% was the linear isomer.

Example 2

A 300 cc Magne-Drive Autoclave was charged with (a) 77.0 g (0.6 mole) of 2-vinyl-4-methyl-1,3-dixoane (VMD) that was treated with $LiAlH_4$ to remove water, 1,3-butanediol and acrolein present therein and then vacuum distilled, (b) 1.57 g (0.005 mole) of excess purified triphenyl phosphite (2% based on the VMD) and (c) 0.00133 g (1.25 x $10^{-6}$ moles) of hexarhodium hexadecacarbonyl [$Rh_6(CO)_{16}$]. The rhodium concentration based on the VMD was 10 ppm. The autoclave was flushed with a 4:1 hydrogen/carbon monoxide mixture and then pressurized to 499,9 kPa (partial pressure of carbon monoxide of 100,0 kPa) and held constant at that value. The gas mixture above the liquid in the autocalve was maintained at a constant ratio (4:1 $H_2$/CO) by purging the gas at a rate of 750 cc/minute. The contents were heated by an electric oven to 105°C and agitated at 1,500 rpm. The progress of the reaction was monitored by taking aliquots every hour and analyzing by g.l.p.c. After 3 hours, 93.5% of the VMD was converted to aldehyde (92.6%) and 2-ethyl-4-methyl-1,3-dioxane (7.4%). Of the aldehyde product, 83% was the linear isomer.

Example 3

A 300 cc Magne-Drive Autoclave was charged with (a) 77.0 g (0.6 mole) of 2-vinyl-4-methyl-1,3-dioxane (VMD) that was treated with $LiAlH_4$ to remove water, 1,3-butanediol and acrolein present therein and then vacuum distilled, (b) 1.57 g (0.005 mole) of excess purified triphenyl phosphite (2% based on the VMD) and (c) 0.00133 g (1.25 x $10^{-6}$ moles) of hexarhodium hexadecacarbonyl [$Rh_6(CO)_{16}$]. The rhodium concentration based on the VMD was 10 ppm. The autoclave was flushed with a 2:1 hydrogen/carbon monoxide mixture and then pressurized to 258,6 kPa (partial pressure of carbon monoxide of 86,2 kPa) and held constant at that value. The gas mixture above the liquid in the autoclave was maintained at a constant ratio (2:1 $H_2$/CO) by purging the gas at a rate of 100 cc/minute. The contents were heated by an electric oven to 105°C and agitated at 1,500 rpm. The progress of the reaction was monitored by taking aliquots every hour and analyzing by g.l.p.c. After 3 hours, 61.5% of the VMD was converted to aldehyde (95.9%) and 2-ethyl-4-methyl-1,3-dioxane (4.1%). Of the aldehyde product, 84% was the linear isomer.

Comparative Example A
*Using tri-n-butyl phosphite as ligand*

A 300 cc Magne-Drive Autoclave was charged with (a) 77.0 g (0.6 mole) of 2-vinyl-4-methyl-1,3-dioxane (VMD) that was treated with $LiAlH_4$ to remove water, 1,3-butanediol and acrolein present therein and then vacuum distilled, (b) 1.27 g of excess tri-n-butyl phosphite (2% based on the VMD) and (c) 0.000532 g (5.0 x $10^{-7}$ moles) of hexarhodium hexadecacarbonyl [$Rh_6(CO)_{16}$]. The rhodium concentration based on the VMD was 4 ppm. The autoclave was flushed with a 15:1 hydrogen/carbon monoxide mixture and then pressurized to 1330,7 kPa and held constant at that value (partial pressure of carbon monoxide was 89,6 kPa). The contents were heated by an electric oven to 105°C and agitated at 1,500 rpm. The gas mixture above the liquid in the autoclave was maintained at a constant ratio (15:1 $H_2$/CO) by purging the gas at a rate of 3.0 1/min. The progress of the reaction was monitored by taking aliquots every hour and analyzing by g.l.p.c. After 3.75 hours, 9.87% of the VMD was converted to aldehyde and 2-ethyl-4-methyl-1,3-dioxane product. The molar ratio of linear to branched isomer was 69/31. The half-order rate constant for this reaction was 0.002 $m^{1/2}$ $min^{-1}$.

Comparative Example B
*Using triphenyl phosphine as ligand*

A 300 cc Magne-Drive Autoclave was charged with (a) 77.0 g (0.6 mole) of 2-vinyl-4-methyl-1,3-dioxane (VMD) that was treated with $LiAlH_4$ to remove water, 1,3-butanediol and acrolein present therein and then vacuum distilled, (b) 1.31 g of excess triphenyl phosphine (1.7% based on the VMD) and (c) 0.000532 g (5.0 x $10^{-7}$ moles) of hexarhodium hexadecacarbonyl [$Rh_6(CO)_{16}$]. The rhodium concentration based on the VMD was 4 ppm. The autoclave was flushed with a 17:1 hydrogen/carbon monoxide mixture and then pressurized to 1172,2 kPa and held constant at that value (partial pressure of carbon monoxide was 69,0 kPa). The gas mixture above the liquid in the autoclave was maintained at a constant

ratio (17:1 $H_2/CO$) by purging the gas at a rate of 4.0 1/min. The contents were heated by an electric oven to 105°C and agitated at 1,500 rpm. The progress of the reaction was monitored by taking aliquots every hour and analyzing by g.p.c. After 5 hours, 30.5% of the VMD was converted to aldehyde and 2-ethyl-4-methyl-1,3-dioxane product. The molar ratio of linear to branched isomer was 68/32. The half-order rate constant for this reaction was 0.005 $m^{1/2}$ $min^{-1}$.

Example 4

The procedure of Comparative Example B was followed except that 1.57 g of excess triphenyl phosphite (2% based on the VMD) was used in place of triphenyl phosphine. After 5 hours, 99.5% of the VMD was converted to aldehyde and 2-ethyl-4-methyl-1,3-dioxane. The molar ratio of linear to branched isomer was 84/16. The half-order rate constant for this reaction was 0.017 $m^{1/2}$ $min^{-1}$.

Example 5

Into a 300 cc Magne-Drive Autoclave was charged under an atmosphere of dry nitrogen, 77.0 g of 2-vinyl-4-methyl-1,3-dioxane (VMD) that was treated with $LiAlH_4$ to remove water, 1,3-butanediol and acrolein and then vacuum distilled, 0.0066 g of hexarhodium hexadecacarbonyl $[Rh_6(CO)_{16}]$ and 1.57 g of excess triphenyl phosphite ligand. The rhodium concentration based on the VMD was 50 ppm. The excess ligand concentration was 2% based on the VMD. The autoclave was flushed with a 1:1 hydrogen/carbon monoxide mixture and then pressurized to 172,4 kPa and held constant at that value (partial pressure of carbon monoxide was 137,9 kPa). The autoclave was heated to 105°C to effect reaction. After 85 minutes, 74% of the VMD was converted to aldehyde and 2-ethyl-4-methyl-1,3-dioxane product. The molar ratio of linear to branched aldehyde product was 86/14.

INDUSTRIAL APPLICABILITY

The hydroformylation of the cyclic acrolein acetals to the corresponding aldehydes under the conditions described herein is especially adaptable to the preparation of aldehydes of cyclic acrolein acetals that can be hydrogenated and hydrolyzed to form 1,4-butanediol which is useful ias a solvent and as an intermediate for the preparation of tetrahydrofuran and various resins.

## Claims

1. The process of hydroformylating cyclic acrolein acetals of the general formulae

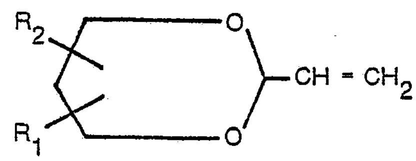

or

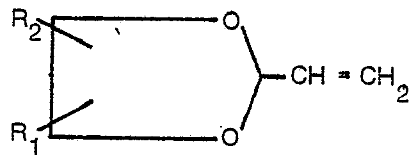

where $R_1$ and $R_2$ are selected from H and the same or different alkyl groups of 1—4 carbon atoms, to form the corresponding aldehydes in the presence of hydrogen, carbon monoxide, rhodium complex catalyst and organophosphorus ligand at a temperature of from 100—115°C, characterized by the presence of 1—50 ppm of rhodium in the complex catalyst based on the cyclic acrolein acetal, a ratio of $H_2$:CO of from 1:1—20:1, a 1—3% by weight based on the cyclic acrolein acetal excess of triphenyl phosphite ligand, and a pressure such that the CO partial pressure is from 34,5—137,9 kPa.

2. The process of Claim 1 wherein the temperature is from 105—110°C.

3. The process of Claim 1 wherein the rhodium concentration is from 1—10 ppm and the ratio of $H_2$:CO is from 2:1—20:1.

4. The process of any of Claims 1—3, wherein the cyclic acrolein acetal is 2-vinyl-4-methyl-1,3-dioxane.

5. The process of any of Claim 1—3, wherein the cyclic acrolein acetal is 2-vinyl-5-methyl-1,3-dioxane.

## Revendications

1. Procédé d'hydroformylation d'acétals cycliques d'acroléine répondant aux formules générales

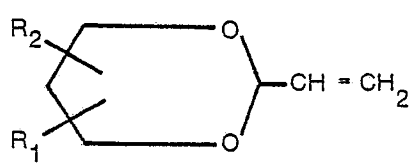

ou

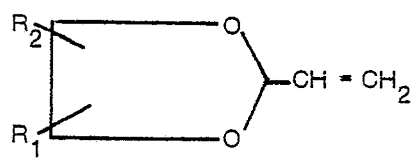

dans laquelles $R_1$ et $R_2$ sont choisies parmi H et des groupes alkyles semblables ou différents de 1 à 4 atomes de carbone, pour la formation d'aldéhydes correspondants, en présence d'hydrogène, de monoxyde de carbone, de complexe de rhodium comme catalyseur et de ligand organophosphore, à une température de 100 à 115°C, caractérisé par la présence de 1 à

50 ppm de rhodium dans le catalyseur complexe relativement à l'acétal cyclique d'acroléine, par un rapport $H_2/CO$ compris entre 1:1 et 20:1, par un excès de ligand phosphite de triphényle de 1 à 3% en poids relativement à l'acétal cyclique d'acroléine, et par une pression telle que la pression partielle de CO est de 34,5 à 137,9 kPa.

2. Procédé selon la revendication 1, dans lequel la température est de 105 à 110°C.

3. Procédé selon la revendication 1, dans lequel la concentration de rhodium est de 1 à 10 ppm et le rapport $H_2/CO$ est compris entre 2:1 et 20:1.

4. Procédé selon l'une quelconque des revendications 1 à 3, dans lequel l'acétal cyclique d'acroléine est le 2-vinyl-4-méthyl-1,3-dioxanne.

5. Procédé selon l'une quelconque des revendications 1 à 3, dans lequel l'acétal cyclique d'acroléine est le 2-vinyl-5-méthyl-1,3-dioxanne.

**Patentansprüche**

1. Verfahren zur Hydroformylierung von cyclischen Acroleinacetalen der allgemeinen Formeln

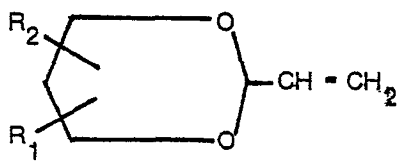

oder

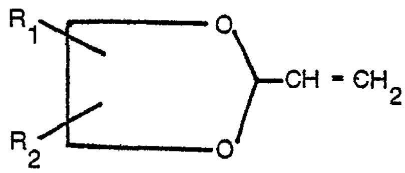

in welchen $R_1$ und $R_2$ ausgewählt sind unter H und gleichen oder verschiedenen Alkylresten mit 1 bis 4 Kohlenstoffatomen, zur Bildung der entsprechenden Aldehyde in Gegenwart von Wasserstoff, Kohlenmonoxid, einem Rhodium-Komplexkatalysator und einem organischen Phosphorliganden bei einer Temperatur von 100 bis 115°C, gekennzeichnet durch die Anwesenheit von 1—50 ppm Rhodium in dem Komplex-katalysator, bezogen auf das cyclische Acroleinacetal, ein Verhältnis von $H_2$:CO von 1:1—20:1, einen Überschuss von 1—3 Gew.-% an Triphenylphosphit-Ligand, bezogen auf das cyclische Acroleinacetal, und einen solchen Druck, dass der CO-Partialdruck 34,5—137,9 kPa beträgt.

2. Verfahren nach Anspruch 1, in welchem die Temperatur 105 bis 110°C beträgt.

3. Verfahren nach Anspruch 1, in welchem die Rhodiumkonzentration 1 bis 10 ppm und das Verhältnis von $H_2$:CO 2:1 bis 20:1 betragen.

4. Verfahren nach einem der Ansrpüche 1 bis 3, in welchem das cyclische Acroleinacetal 2-Vinyl-4-methyl-1,3-dioxan ist.

5. Verfahren nach einem der Ansprüche 1 bis 3, in welchem das cyclische Acroleinacetal 2-Vinyl-5-methyl-1,3-dioxan ist.